# EUROPEAN PATENT APPLICATION

(11) **EP 1 430 852 A2**
(43) Date of publication of application: **23.06.2004**
(21) Application number: 03090443.7
(22) Date of filing: 17.12.2003
(51) Int. Cl.: A61C 13/00, A61F 2/30

(54) **Method for computer controlled machining of customized medico-dental parts and blank for manufacturing prosthetic components**

(30) Priority: 19.12.2002 BR 0205696; 03.06.2003 US 452727
(71) Applicant: Biogénie Projectos Ltda., Rio de Janeiro, RJ 20071-000 (BR)
(72) Inventor: Branco de Luca, Silvio Castello, Leblon Rio de Janeiro, RJ (BR); de Andrade de Mello e Souza, Eduardo Cezar, Barra de Tijuca Rio de Janeiro, RJ (BR); de Lima Spinola, Alexandre, Copacabana Rio de Janeiro, RJ (BR); da Rocha Alburquerque, Eduardo, Lagoa Rio de Janeiro, RJ (BR); Ladeira Casado, Mauricio, Sao Lourenco Niteroi, RJ (BR)
(74) Representative: Eisenführ, Speiser & Partner

(57) **Abstract**

A customized prosthesis, or instrument, for medical/dental applications is provided which replicates the desired bone-graft, tooth, or tool, being replaced. The dimensions of the prosthesis, or instrument, are determined by mathematically interpolating key-points that characterize a specific part. A computer controlled machine then cuts the desired part out of a pre-fabricated blank, directly at the site of operation. Methods of the invention relate to selecting the type of part being replaced, identifying and measuring the coordinates of key-points for that part, and initializing the automated machining process. Also, special supporting devices that include pre-fabricated features common between certain parts, are used in order to facilitate the machining process. The identification of key-points is done by comparing a schematic drawing of the type of part being replaced to the actual part. A grid is then used to measure the coordinates for those key-points.

## Description

### FIELD OF THE INVENTION

The present invention relates to customized instruments and parts and methods for producing such customized instruments and parts. More specifically, the invention relates to customized instruments and parts fabricated through the use of computer-controlled machinery, directly at the location where these pieces will be used. Also presented here are blanks, supporting devices with blocks of raw material from which a custom piece is cut, used to facilitate the machining of implant fixtures.

This invention is presented with text and examples relating to the field of dentistry, as it represents the preferred implementation of this method, but those skilled in the art will know how to apply the present method to produce other customized instruments and parts.

### BACKGROUND OF THE INVENTION

Prosthetic restorative systems seek to provide functional and cosmetic replacements for missing body parts. When a patient looses part of his dentition, it is up to the dentist to select how to best substitute the lost part. Normally a bridge can be used, in this case the surrounding teeth anchor a replacement for the lost tooth. Dental implants are used when the patient, for various reasons, requires a prosthetic device to hold one or more artificial teeth in place. For example, the teeth surrounding the portion requiring the prosthesis may be too weak or far apart to provide adequate strength for bridging. A dental implant is usually divided into three components. These are the implant fixture, the abutment, and the crown. The fixture anchors the prosthetic components to the bone, providing both support and stability to the implant. The crown must exactly replicate the characteristics of the dentition being replaced. In order to do so, crowns are usually made with a ceramic material, which, due to its fragility, is limited to a few hundredths of an inch in thickness. Due to these size limitations, the abutment is used to provide a link between the crown and the implant fixture, it also holds the crown in proper alignment relative to the implant fixture and absorbs the stress of chewing. A customized abutment should also match the size, shape and contour of the original tooth in order to provide the best possible appearance.

It can be very time consuming to fit a fixture into the bone and sit an abutment over it that matches the ideal position for the crown. After the initial treatment there might be complications with the quality of the remaining bone, forcing the dentist to angle the fixture in order to anchor it in solid bone. These angles compound the challenge in making a customized abutment. Abutments can also be difficult to seat on the implant the later in the process they are placed, resulting in the entrapment of the gums before the crown can be put into place. Once the abutment is placed, several X-rays may be required to ensure that it is properly seated, resulting in more time and expense.

The standard process of providing a prosthetic replacement for a patient involves specialized labor and requires several visits to the dentist's office. An oral surgeon or periodontist is required to implant the dental implant fixture in the patient's jawbone. A technician is normally responsible for manufacturing study models and final prosthetic parts. Finally, a general dentist or prosthodontist performs the fitting of the prosthesis. During the process, the patient must make several visits to the dentist's office so that all steps in the treatment may be carried out. These steps include the casting of a study model, wax modeling of the prosthesis, the manufacture of surgical guides, surgery for installing the implant fixture, exposure of the implant fixture in order to place a temporary healing crown, casting of the situation model, wax modeling the abutment, manufacturing the abutment, testing and adjusting the abutment, casting the crown and installing both abutment and crown.

A computerized system could be used to simplify most of the steps and personnel included in the standard process of providing a prosthetic replacement, however, no satisfactory manufacturing system for customized prosthetic replacements is believed to exist to permit on-site inspection, design and machining of a prosthetic replacement that is at the same time fast, flexible and cost-effective. Doctors currently use either (i) pre-fabricated off-the-shelf parts, which, although readily available, cannot be customized thus rarely fulfilling the patients' specific need; (ii) hand-made custom parts, which take a long time to produce and are subject to the imperfections, high-cost, and need for special-training of manual labor; (iii) on-site machine-made custom parts which are expensive and limited to manufacturing only one or two kinds of prosthetic parts, usually crowns, fillings or bridges; or (iv) off-site machine-made custom parts, which solve the problem of preparing custom parts, but require large factories and equipment, take a long time to prepare, need specially trained labor, dedicated machinery and include costs outside the normal treatment scope (such as shipping, customs, and factory downtime).

In what regards the computerized manufacturing systems, there is an option that assembles to waxing the flexibility of computer design techniques. In the Procera system (for example) the initial mold prepared with wax is digitalized using a point mapping system, following which an operator can check and alter the points of the model by computer before sending it electronically to the central manufacturing installation. At the manufacturing installation, large machines controlled by computer manufacture the part, which is then returned by mail to the dentist's office.

The Procera system is one of the possible applications for CAD/CAM (Computer Aided Design/Manufacture) technology in the field of dentistry. Another example was developed by Atlantis Components Inc., whereby instead of digitalizing the whole mold to the computer, the technician simply inserts key measurements that will allow the computer to modify a previously stored version of the type of part to be manufactured, obtaining a digital model.

Patents US 6,231,342 and US 5,989,029, both in the name of Atlantis Components Inc., respectively claim a customized dental abutment having dimensions determined by a computer algorithm that modifies, through the input of specific measurements, standard pre-programmed part shapes; and, a method for the selection of dental abutment components from a computerized data bank. In both patents the model generated by computer is then used to make a prototype of the customized abutment component, preferentially in wax.

It is worth pointing out that despite Atlantis having encountered solutions for design difficulties by developing standard tooth shapes and storing them in computer, their method still present inconveniencies such as the necessity of computers capable of processing a large data bank of parts.

An undesirable characteristic common to the latter method is the fact that the installation where the milling takes place is remote from the site of operation.

The current solutions therefore require considerable investments in labor, machinery, and items outside the normal scope of the dentist's office. The many visits required to treat the patient also mean these solutions diminish the number of patients a dentist can treat. Typically, dentists will stock different kinds of parts, in an attempt to save some of the time needed to treat a patient, this however is not a welcome investment given such parts' uncertainty of use. Accordingly, there remains a need in the art for methods and materials that will aid in reducing the time, labor and cost of dental implant restorations. The present invention addresses this need by presenting a method for on-site computer controlled machining of prosthetic devices, that is at the same time fast, flexible, easy-to-use and cost-effective, and utilizes pre-fabricated blanks to further facilitate the manufacturing process.

### SUMMARY OF THE INVENTION

The present invention relates to a method for providing prosthetic replacements that replicate the same characteristics and functions of the lost parts. The key improvement on the available art, is the ability to manufacture on-site a wide range of prosthetic components, no longer limited to only altering dimensions, nor dependent of high-cost machinery and specially trained operators.

Recent advances in the fields of micro mechanics and machine control now allow for the creation of small, computer controlled machines that are at the same time quiet, fast and cost-effective enough to be deployed inside a dentist's office. Software technology also contributed once it allowed for complex operations to be automatically executed and displayed as easy to use visual information, thus eliminating the need for special machine operation training.

Prosthetic components of the present invention are customized to replicate a body part being replaced. A list is presented so that the user may select the kind of prosthetic replacement to be machined. Once the selection has taken place, key-points of the part being replaced are extracted from a model, CT or equivalent digital scanner. Said key-points are then processed using a computer algorithm to generate a complete on-screen 3D model of the prosthetic replacement. Adjustments can be made to the computer model before machining the final piece. After the finalized on-screen model is approved by the user, it is converted into machine code. Finally, the user is prompted to insert the proper blank prior to activating the machining process.

The technology developed to provide the present invention also allows for other kinds of prosthetic components to be customized thus replicating a body part being replaced. These include, but are not limited to: a section of the skull; a section of the mandible; a section of the calvaria; a section of the, or the entire, clavicle; a section of the, or the entire, scapula; a section of the, or the entire, sternum; a section of the, or the entire, humerus; a section of the, or the entire, rib; a section of the, or the entire, pelvis; a section of the, or the entire, radius; a section of the, or the entire, ulna; a section of the, or the entire, carpus; a section of the, or all the, metacarpal bones; a section of the, or all the, phalanges (fingers and toes); a section of the, or the entire, femur; a section of the, or the entire, patella; a section of the, or the entire, tibia; a section of the, or the entire, fibula; a section of the, or the entire, tarsus; a section of the, or the entire, spinal column. Medical-dental parts also comprise dental implant components, tool handles, dental and coronary prosthetic components such as implant fixture components, dental abutments components and dental crowns.

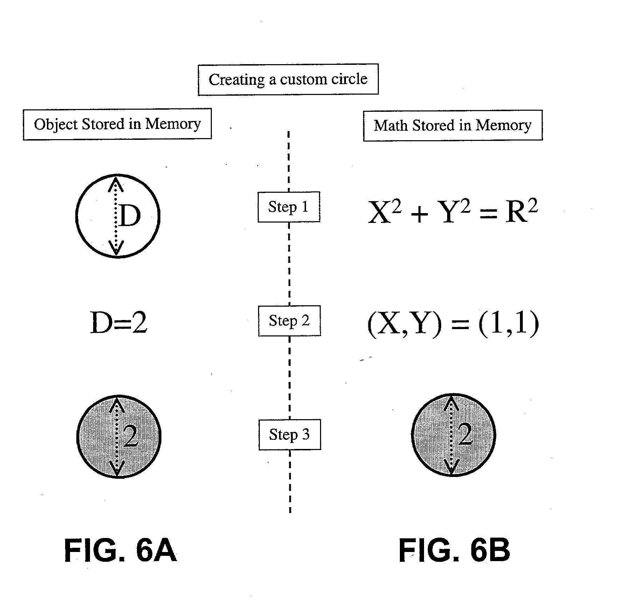

The initial steps of selecting a prosthetic replacement from a list and extracting key-point for that part, relate to the computer algorithm used to generate the on-screen 3D model. In the method proposed by the present invention, no object is previously stored in the computer's memory, only the algorithms capable of generating such object from key-points of the object are stored. This is detailed further in FIG. 6 where the different techniques for mapping objects are compared.

Once said key-points have been extracted and a 3D on-screen model generated, the information is presented to allow the user to make modifications to the proposed part. When satisfied with the modified part other information such as the type of material to be cut must be input by the user. The information might also be saved at any time so that the work may later continue or be repeated.

According to the invention, after the desired part is designed to satisfaction, another computer algorithm is employed to convert the information into machine code. This code is normally stored as activation instructions for the individual motors in the machine (such as speed, time, and direction of activation), however this information may also be stored as common CNC machine code (such as G-code).

After finalizing the above processes a part is now ready to be machined. A final method of the invention relates to the command and control of the automated machining process, wherein the stored machine code that characterizes the part is checked for any errors, the user is prompted to insert the proper blank from which the part will be cut, the blank checked and used to calibrate the machine, finally the part is cut then cleaned for immediate decontamination and surgical installation.

In short, the present invention relates to a method for on-site computer controlled machining of customized medical-dental parts from pre-fabricated blocks of raw material comprising the following steps: (i) selection of the part being replaced, (ii) selection between manual or automated identification of said part's key-points, (iii) selection between manual or automated measurement of said key-points' coordinates, (iv) use of a computer algorithm that employs mathematical interpolation of said key-points to calculate the dimensions and generate a virtual model of said part, (v) use of a computer algorithm that gives the user visual feedback on said model by displaying it onscreen, (vi) optionally, manually adjustment the dimensions of said part in said model, (vii) convert said model to corresponding machine code, (viii) selection of type of block of raw material to be used, (ix) insertion of said block of raw material in the machine, (x) use of a computer algorithm to confirm if said block of raw material is appropriate to machine said machine code, and (xi) use of a computer algorithm to calibrate and control the automated machining process of said machine code to produce said part.

The present invention will greatly simplify the treatment and process of installing a prosthetic component, where instead of the many visits to the dentist's office described earlier, for example, the patient might have only three visits. These would be, one for planning and casting study models, one for installing the implant fixture custom abutment and healing crown, and a third to check the condition of the patient and install the final crown. In this scenario, the custom abutment would be milled on-site, during the final stages of surgical procedure. The reduced number of visits has two direct consequences, the cost of the treatment will be reduced for the patient and the dentist will be able see more patients in a given period. Other consequences include the reduction of unnecessary parts in stock since only blanks will have to be stocked whose consumption is assured given their flexibility of use.

Another embodiment of the present invention relates to a blank for manufacturing prosthetic components, comprising support device and block that allow customized dental fixtures to be machined, wherein the block to be machined (12) possesses an anti-rotational device (31) and an internal thread (14) located in one of the faces, which allows fixation by stud to the support element (13); this support element (13) having a seating area at one extremity that contains a matching insert for the anti-rotational device (31) and a continuous passage (15) that allows the fixing stud access to the block (12), with the support also possessing an external body geometry that serves as a guide to insert it appropriately in the milling machine.

### DESCRIPTION OF THE DRAWINGS

Other objects and advantages of the present invention will become more apparent upon reference to the following detailed description and annexed drawings in which:
- FIG. 1a and 1b: present the entire dental prosthetic replacement as seen from its sagittal and frontal planes;
- FIG. 2a and 2b: present examples of blanks to be used in the manufacturing of customized dental implant parts;
- FIG. 3a and 3b: illustrate the machining process that will be performed in order to create the final piece from the original blank;
- FIG. 4: schematically represents the proposed method;
- FIG. 5: shows currently available off-the-shelf prosthetic replacements as a reference point to compare the custom parts;
- FIG. 6a and 6b: illustrate the different information needed to generate customized objects from (a) a previously stored object whose measurements are altered, and (b) using the object's key-points with its mathematical relations to generate the custom model;

### DETAILED EMBODIMENT

The present invention relates to methods and materials used in the manufacturing of prosthetic replacements. Initially, the doctor must choose the kind of problem he wishes to solve (i.e. which part of the body will be prothetically replaced). Once this is done, the characteristics of the desired part must be informed to the system (manually or digitally by means of a model scanner), so that it can then be viewed on-screen and modified in the computer before proceeding to be machined by a small CNC machine at the actual site of operation.

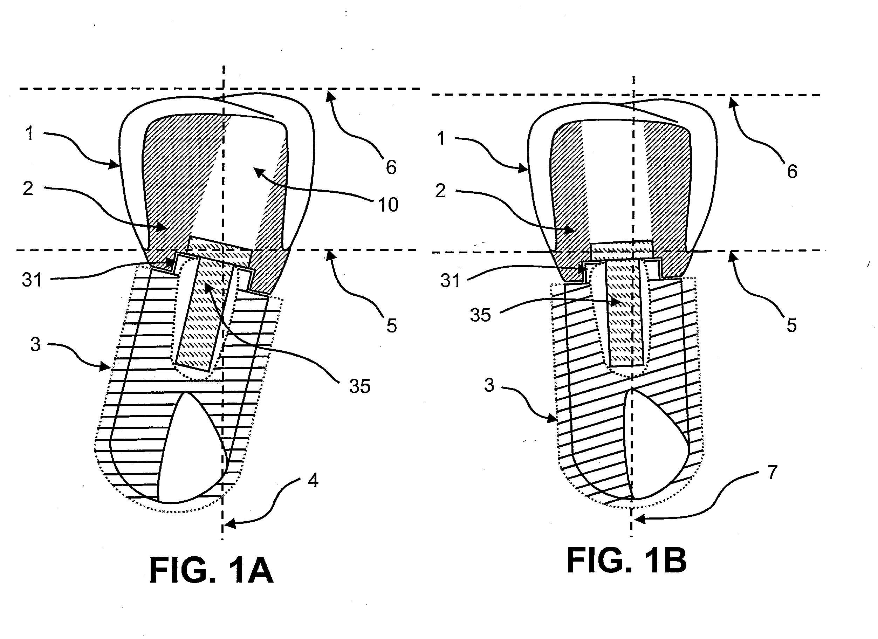

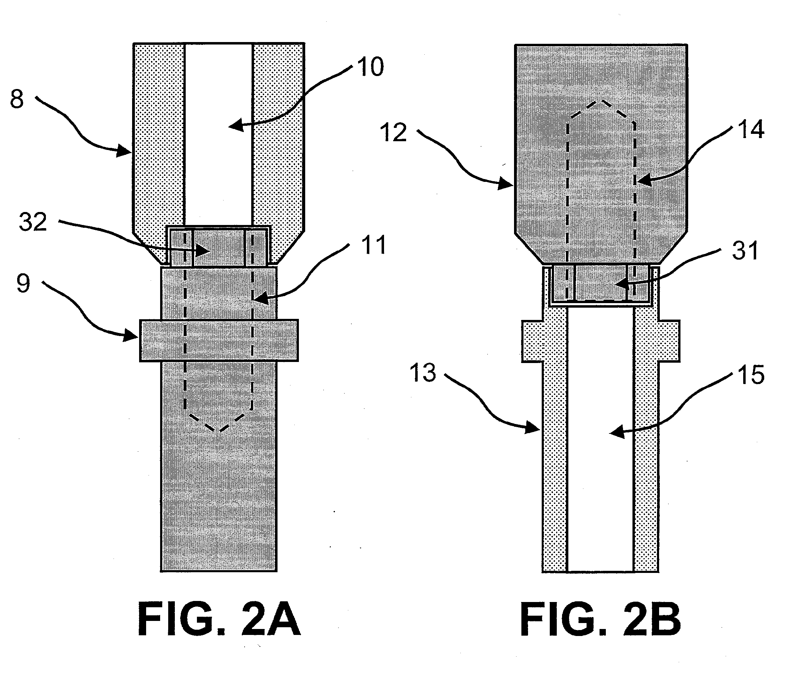

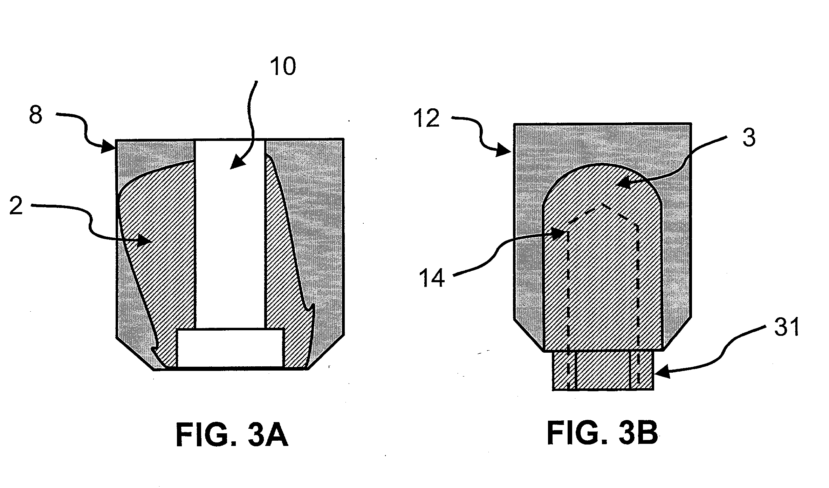

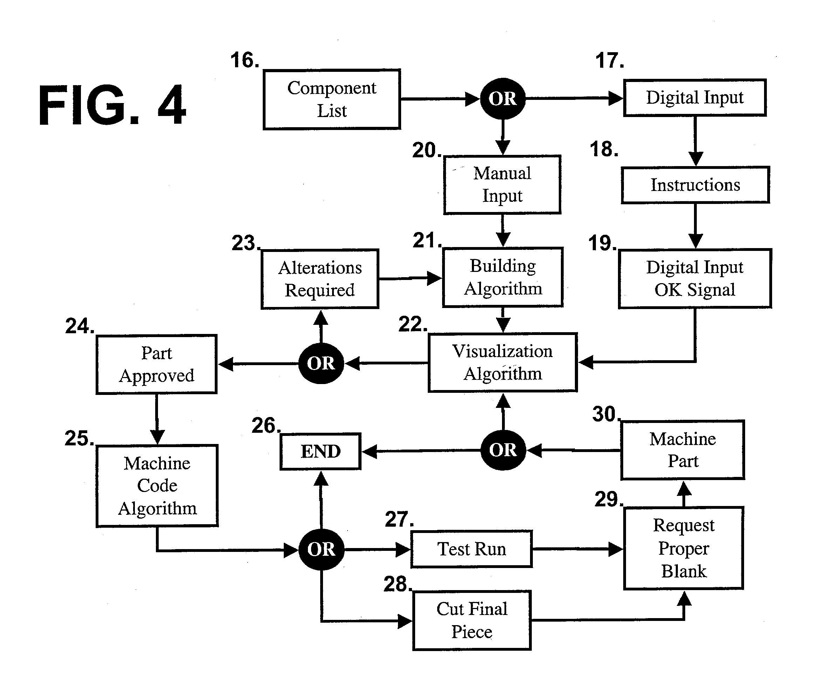

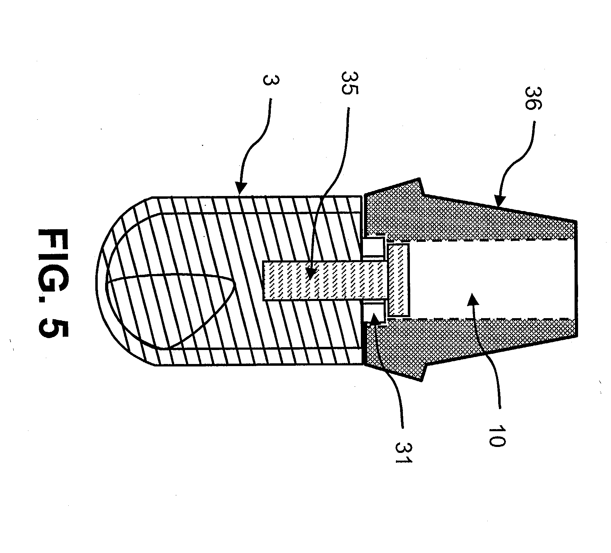

Briefly, FIGS 1 a and 1b represent one instance of objects to be manufactured, FIGS 2a and 2b show the blanks that are to be transformed during the process, FIGS 3a and 3b the cutting that will be done and FIG 4 shows a diagram of how the process occurs. FIG 5 relates to the existing technique where a pre-fabricated abutment component 36 is used, while FIGS 6a and 6b compare techniques for data storage in computers.

FIG 1 a presents the entire implant viewed from the sagittal plane, while FIG 1b shows the frontal plane view. In these Figures it is possible to observe the crown 1 which is the cap that covers the dental prosthesis, the abutment 2 which is the structural element of the prosthesis, and the implant fixture 3 which is the element that anchors the joint components to the jawbone of the patient. FIG 1a also shows the frontal plane 4 (note that this is orthogonal to the view of the figure), while 3 the sagittal plane 7 can be seen in FIG 1b (also orthogonal to the view shown). The crown is fixed to the abutment with chemical adhesives, but the abutment is fixed to the implant fixture by means of a support stud 35 that holds the anti-rotational element 31 of the fixture against its matching insert in the abutment.

Two other planes used as alignment guides are also represented in FIGS 1a and 1b. These are the occlusal plane 6 that is indicative of the top level of the teeth and the gumline 5 that is indicative of the level of the gums around the tooth.

FIG 2a shows the raw part ready to be transformed into a customized abutment. Composed of the element held in the machine for support 9 and the raw part to be milled 8 whose material may be metallic, ceramic, wax, or a biomaterial. Note that to enable a connection there exists a passage 10 through which the support stud 35 will pass, with the support element 9 requiring an internal thread 11 to take the stud.

FIG 2b shows a raw part ready to be transformed into a customized implant fixture, composed of an element held in the machine for support 13 and a raw part to be milled 12. The part to be milled 12 may be a metallic, ceramic, wax or biomaterial type material. An example of metallic material would be titanium (presently being successfully used in implants), while biomaterials (considered the material of the future in the field of prostheses) include human or bovine Demineralized Freeze Bone Dry Allograft (DFBDA); human or bovine Freeze Bone Dry Allograft (FBDA); and also, synthetic or coral hydroxyapatite, amongst others known to those skilled in the art.

Note that for fixing purposes, the implant fixture 3 contains an internal thread 14 that takes the support stud 35, and for such, the support 13 requires a continuous passage 15 through which the stud will pass. Preferably, the fixing stud 35 is of the bolt type. The support 13 also possesses an external body geometry that serves as a guide to insert it appropriately in the machine. More particularly, the support element possesses a seating area at one extremity that contains a matching insert for the anti-rotational device 31. In both cases the anti-rotational element of the implant fixture 31 and the abutment 32 are ready beforehand, together with their matching inserts, in the respective raw parts.

The anti-rotational device located in one of the faces of the block 12 possesses a protrusion, with an equivalent recess being located in the support part 13. The protrusion of the anti-rotational device may have various configurations, the most common being a solid hexagon or a Morse cone. Preferably, the protrusion consists of an elevated part configured as a thin disk of larger diameter and a superimposed hexagonal nut having a smaller limiting diameter.

The cutting process to be performed is represented in FIGS 3a and 3b, respectively showing the raw part 8 being reduced by machine to the desired dimensions of the abutment 2 component, and the raw part 12 also reduced by machine to the desired dimensions of the implant fixture 3 component. Note the passage 10 for the connecting stud as well as the internal thread 14 to retain the stud in the implant fixture are already present in the raw parts.

The schematic diagram for the method is presented in FIG 4 and comprises the following stages:

Initially 16 the component to be manufactured is selected from a list of types presented in the system, after which the operator can choose to provide the characteristics of the desired part manually 20 or digitally 17. In the case the operator chooses to digitalize the desired part 17, the system will then provide text instructions 18 on how to proceed.

The method is not limited to any particular type of digitalizing device, however it is necessary that the information supplied by the digitalizing source be compatible with the kind of information (points and coordinates) the system needs in order to generate a model. Devices that may be used range from simple digital cameras to modern magnetic resonance devices.

The digitalization ends when the system recognizes the data provided and informs by means of a positive signal 19 that the operation may continue.

The manual entry 20 offers the doctor an option in case he does not possess (or wish to use) a digitalizing device. In this sequence, the system asks the user to inform characteristic key-points by means of a building algorithm 21 which will employ the mathematical ratios of the part, stored in memory, to build the desired digital model.

After either manual or digital entry, a visualization algorithm will present the user with the part to be machined for approval 24 or alteration 23.

Once the model is approved the system converts it into machine code through a computer algorithm 25, after which, the part may be manufactured as a wax prototype 27, as a final product 28 or simply stored in memory for later use 26.

If machining is selected the system activates the actuators within the CNC machine and asks the user to insert the correct blank with the appropriate dimensions and material 29. After having checked the correct raw part has been inserted, the system then goes through a calibration routine following which it begins machining the part 30. Once the manufacturing has finished the system returns to the visualization mode to enable the user to make modifications or simply terminate the operation.

In detail, the method of the present invention for the design and production of customized objects begins with the user (in this case the doctor or his assistant) selecting from the system the type of object required from a list of pre-determined objects. The system then presents a list of subcategories and awaits the choice of one item by the user.

After this, the user must provide the initial characteristics of the object by defining a fixed number of points spatially distributed or start the digitalizing device. The process of digitally registering the cast of the dental arcade is a process that must have occurred before the entry of the data referring to the spatial points. They are, however, independent and mutually exclusive processes. In other words, an option must be made. They can be linked if a more complete set of data is generated, which is the ideal for milling the final part. The characteristics of the object, such as material, shape and type of finish can then be seen on the screen for any immediate adjustment. Therefore, after generating a primary object from the data provided by the user, the object is shown for visualization and adjustment.

Different from that which is presented in patent US 6,231,342, no object is previously stored in the computer memory. Only the computer algorithm capable of generating the desired part from spatial points, or from the digitalizing device, is stored. In this manner, only the mathematical ratio derived from the objects is stored, which reduces the disk space necessary to store the types of instruments or medical parts. This simplification also improves the quality of the service, and also the updating of the system, where compact algorithms of mathematical ratios can be sent electronically to the user in the case a part not present on the initial list is required.

Mathematical ratios are used, because they occupy less space, thus allowing persons with less powerful computers to operate the software and control a CNC machine.

When creating the algorithm, one of the greatest difficulties refers to the representation of the mathematical ratios between the surfaces, or, in other words, finding the equations representative of the figures. This problem was solved through the spatial interpolation of bi-dimensional curves, in the following manner:
(i) The entire part has a measurable number of critical points, with characteristics of maximum and minimum unique for a given type of geometry;
(ii) The lateral profiles of the parts can be equated applying the techniques of regression to the critical points measured. Thus it is possible to obtain the equations for the bi-dimensional curves characteristic to that part;
(iii) The quantity of curves used depends on the required finish of the model but this can be pre-determined and therefore does not present a problem;
(iv) Finally, the computer model can be built applying the techniques of spatial interpolation to the bi-dimensional curves.

The solution developed by the present applicants employs the stored equations together with the new critical points (with measurements taken from the patient) to generate a model of the parts. Following which the models may be adjusted by operators (doctors or assistants) to the desired shape. Measurements may be taken manually, or by means that include, amongst others, image registering devices (optical) and magnetic resonance devices. Whatever the instrument, the dimensions of the patient must be measured and then inserted in the computer.

Computer techniques that may be employed in the acquisition of measurements include, but are not limited to, computer vision techniques such as the processing, enhancement and segmentation of images. The adjustment of the model is done using 3D modeling techniques and graphic imaging, allowing dentists and assistants to use the system through a graphic interface, thus dispensing with any training in specific techniques.

The objects manufactured using the present invention may vary in shape, material and height. When considering dental implant components as objects, a series of measurements at the location of the tooth are undertaken, including: measurements of the angles of the elements of the implant in reference to the sagittal (7), frontal (4) and occlusal (6) planes and gum profile (5); measurement of the spaces to be occupied by the prosthesis, also on the sagittal (7), frontal (4) and occlusal (6) planes and gum profile (5); verification in reference to these same planes of the positioning of the anti-rotational element (31) already installed in the location.

Measurements of any or all these parameters are aimed at obtaining the critical points of the part to be made. In a preferred embodiment of the present invention, these parts are either an abutment component very close in size, shape and position to the tooth being substituted, or a crown to act as a cap, or an implant fixture to anchor the prosthesis. The initial choice of the type of tooth being substituted (for example, lateral incisor, first pre-molar, etc.), dimensions obtained and adjustments made are then processed by the system to prepare the customized implant element.

Once the user is satisfied with the characteristics of the object, he may commence the production process. A computer algorithm then converts the computer model into a machine code for milling, which remains in readiness until the raw part to be milled is inserted into the machine when manufacturing can commence.

Finally, a CNC machine is employed to physically produce the dental implant components referred to in this present invention. This type of CNC milling tool is known to specialists in the technique and may include, but is not limited to, the producers (and model types): ERGOMAT (TB 42/60 CNC); UNION-BOHRWERKE (UNION T100); SHERLINE (2010 CNC); and KONDIA (K600). The computer controlled machine used in the method of the present invention is not limited to a computer controlled mill as other machines such as a lathe or a computer controlled spark-erosion machine may also be used. In essence, any machine that can be used to manufacture metallic parts may be applied.

This invention is not limited to the embodiment shown above by way of example, but can be modified within the scope of the appended patent claims and inventive concept.

## Claims

1. Method for on-site computer controlled machining of customized medical-dental parts from a pre-fabricated block of raw material comprising the following steps: (i) selection of the part being replaced, (ii) selection between manual or automated identification of said part's key-points, (iii) selection between manual or automated measurement of said key-points' coordinates, (iv) use of a computer algorithm that employs mathematical interpolation of said key-points to calculate the dimensions and generate a virtual model of said part, (v) use of a computer algorithm that gives the user visual feedback on said model by displaying it onscreen, (vi) optionally, manually adjustment the dimensions of said part in said model, (vii) convert said model to corresponding machine code, (viii) selection of type of block of raw material to be used, (ix) insertion of said block of raw material in the machine, (x) use of a computer algorithm to confirm if said block of raw material is appropriate to machine said machine code, and (xi) use of a computer algorithm to calibrate and control the automated machining process of said machine code to produce said part.

2. Method according to claim 1 wherein only the mathematical ratios of the parts able to be milled by the system, are stored in the computer memory.

3. Method according to claim 1 wherein said computer controlled machine comprises a computer controlled mill.

4. Method according to claim 1 wherein said computer controlled machine comprises a computer controlled lathe.

5. Method according to claim 1 wherein said computer controlled machine comprises a computer controlled spark-erosion machine.

6. Method according to claim 1 wherein the automated identification device is a camera for the acquisition of digital images.

7. Method according to claim 1 wherein the automated identification device is a system for tracking and acquiring spatial points, such as a magnetic resonance device.

8. Method according to claim 1 wherein said customized medical-dental part comprises dental implant fixture components.

9. Method according to claim 1 wherein said customized medical-dental part comprises dental abutment components.

10. Method according to claim 1 wherein said customized medical-dental part comprises dental crowns.

11. Method according to claims 1 and 9 wherein the manufacture of the abutment element is carried out using a raw part already containing an anti-rotational device, pre-fabricated into the block to be milled.

12. Method according to claims 1 and 8 wherein the manufacture of the dental fixture component is carried out using a raw part already containing an anti-rotational device, pre-fabricated into the block to be milled.

13. Method according to claim 1 wherein said customized medical-dental part comprises a prosthetic substitute to a section of the skull.

14. Method according to claim 1 wherein said customized medical-dental part comprises a prosthetic substitute to a section of the Mandible.

15. Method according to claim 1 wherein said customized medical-dental part comprises a prosthetic substitute to a section of the Calvaria.

16. Method according to claim 1 wherein said customized medical-dental part comprises a prosthetic substitute to a section of the, or the entire, clavicle.

17. Method according to claim 1 wherein said customized medical-dental part comprises a prosthetic substitute to a section of the, or the entire, scapula.

18. Method according to claim 1 wherein said customized medical-dental part comprises a prosthetic substitute to a section of the, or the entire, sternum.

19. Method according to claim 1 wherein said customized medical-dental part comprises a prosthetic substitute to a section of the, or the entire, humerus.

20. Method according to claim 1 wherein said customized medical-dental part comprises a prosthetic substitute to a section of the, or the entire, rib.

21. Method according to claim 1 wherein said customized medical-dental part comprises a prosthetic substitute to a section of the, or the entire, pelvis.

22. Method according to claim 1 wherein said customized medical-dental part comprises a prosthetic substitute to a section of the, or the entire, radius.

23. Method according to claim 1 wherein said customized medical-dental part comprises a prosthetic substitute to a section of the, or the entire, ulna.

24. Method according to claim 1 wherein said customized medical-dental part comprises a prosthetic substitute to a section of the, or the entire, carpus.

25. Method according to claim 1 wherein said customized medical-dental part comprises a prosthetic substitute to a section of the, or all the, metacarpal bones.

26. Method according to claim 1 wherein said customized medical-dental part comprises a prosthetic substitute to a section of the, or all the, phalanges (fingers and toes).

27. Method according to claim 1 wherein said customized medical-dental part comprises a prosthetic substitute to a section of the, or the entire, femur.

28. Method according to claim 1 wherein said customized medical-dental part comprises a prosthetic substitute to a section of the, or the entire, patella.

29. Method according to claim 1 wherein said customized medical-dental part comprises a prosthetic substitute to a section of the, or the entire, tibia.

30. Method according to claim 1 wherein said customized medical-dental part comprises a prosthetic substitute to a section of the, or the entire, fibula.

31. Method according to claim 1 wherein said customized medical-dental part comprises a prosthetic substitute to a section of the, or the entire, tarsus.

32. Method according to claim 1 wherein said customized medical-dental part comprises a prosthetic substitute to a section of the, or the entire, spinal column.

33. Method according to claim 1 wherein said raw material comprises titanium.

34. Method according to claim 1 wherein said raw material comprises a ceramic type material.

35. Method according to claim 1 wherein said raw material comprises a wax type material.

36. Method according to claim 1 wherein said raw material comprises a biomaterial.

37. Method according to claim 1 wherein said customized medical-dental part comprises dental implant components and tool handles.

38. Method according to claim 1 wherein said customized medical-dental part comprises dental and coronary prosthetic components.

39. Blank for manufacturing prosthetic components, comprising support device and block that allow customized dental fixtures to be machined, wherein the block to be machined (12) possesses an anti-rotational device (31) and an internal thread (14) located in one of the faces, which allows fixation by stud to the support element (13); this support element (13) having a seating area at one extremity that contains a matching insert for the anti-rotational device (31) and a continuous passage (15) that allows the fixing stud access to the block (12), with the support also possessing an external body geometry that serves as a guide to insert it appropriately in the milling machine.

40. Blank according to claim 39 wherein the fixing stud is of the bolt type.

41. Blank according to claim 39 wherein the anti-rotational device (31) located in one of the faces of the block (12), possesses a protrusion, with an equivalent recess in the support part (13).

42. Blank according to claim 41 wherein the protrusion is in the form of a hexagon or a Morse cone.

43. Blank according to claim 42 wherein the protrusion consists of an elevated part configured as a thin disk of larger diameter and a superimposed hexagonal nut having a smaller limiting diameter.
